# EUROPEAN PATENT APPLICATION

(11) **EP 3 892 995 A1**
(43) Date of publication of application: **13.10.2021**
(21) Application number: 19893721.1
(22) Date of filing: 06.08.2019
(51) Int. Cl.: G01N 31/12

(54) **ANALYSIS DEVICE AND ANALYSIS METHOD**

(30) Priority: 04.12.2018 JP 2018227541
(71) Applicant: HORIBA, Ltd., Kyoto 601-8510 (JP)
(72) Inventor: YAMADA, Takahiro, Kyoto-shi, Kyoto 601-8510 (JP); YONESHIGE, Hidenaru, Kyoto-shi, Kyoto 601-8510 (JP)
(74) Representative: Isarpatent
(86) International application number: PCT/JP2019/030902
(87) International publication number: WO 2020/115946

(57) **Abstract**

In order to promote combustion of a sample in a high temperature range and reduces a combustion amount of the sample in a low temperature range, the present invention includes an electric furnace 3 into which a container 2 accommodating a sample W is introduced, a gas analyzer 4 configured to analyze a gas generated from the sample W that is heated and burned in the electric furnace 3, a mixed gas supply path L6 configured to supply, to the electric furnace, a mixed gas obtained by mixing a dilution gas with a combustion-supporting gas that promotes combustion of the sample W, and a concentration changing mechanism 7 configured to change a concentration of the combustion-supporting gas in the mixed gas.

## Description

### Technical Field

The present invention relates to an analysis device and an analysis method for analyzing elements, such as carbon (C) and sulfur (S) included in a sample.

### Background Art

As this type of analysis device, Patent Literature 1 discloses an analysis device in which a container (boat) accommodating a sample is introduced into an electric furnace, and a gas generated from the sample heated and burned in the electric furnace is analyzed.

In such an analysis device, a type of analysis device that analyzes a sample that is difficult to burn such as steel or non-ferrous metal (hereinafter, also referred to as a flame-retardant sample) is configured to supply, for example, a high-concentration oxygen gas having a concentration close to 100% to inside of an electric furnace as a combustion-supporting gas, and to promote combustion of the sample.

However, when an easily combustible sample (hereinafter, also referred to as an explosive sample) is analyzed by such an analysis device, the amount of gas generated by rapid combustion of the sample is large, a peak of a signal detected by a gas analyzer becomes steep, and there arises a problem that accurate analysis cannot be performed unless a detector has a high resolution. Moreover, if the explosive sample is scattered due to rapid combustion, this also causes a decrease in analysis accuracy and further requires maintenance such as cleaning of the inside of the electric furnace.

There are also problems as follows.

That is, in analysis of a mixed sample including a component that burns in a low temperature range and a component that burns in a high temperature range using the above analysis device, the component that burns in the low temperature range and the component that burns in the high temperature range included in the mixed sample can be separated and analyzed by, for example, providing the electric furnace with a function of increasing the temperature and increasing the temperature in the electric furnace.

However, if a high-concentration oxygen gas is supplied to the electric furnace as described above, the amount of combustion of the component that burns in the low temperature range becomes large, and an oxidation heat generated by the combustion causes the component to be burned in the high temperature range to burn in the low temperature range. Thus, there is a concern that the analysis accuracy of separate analysis may decrease.

### Citation List

### Patent Literature

Patent Literature 1: JP 2952703 B2

### Summary of Invention

### Technical Problem

The present invention has been made to solve the above problems at once, and a main object of the present invention is to flexibly change an amount of combustion in accordance with a type of sample, promote combustion, suppress rapid combustion, and achieve analysis of various samples.

### Solution to Problem

That is, an analysis device of the present invention includes an electric furnace into which a container accommodating a sample is introduced, a gas analyzer configured to analyze a gas generated from the sample that is heated and burned in the electric furnace, a mixed gas supply path configured to supply, to the electric furnace, a mixed gas obtained by mixing a dilution gas with a combustion-supporting gas that promotes combustion of the sample, and a concentration changing mechanism configured to change a concentration of the combustion-supporting gas in the mixed gas.

The analysis device configured in this way allows the concentration of the combustion-supporting gas in the mixed gas to be changed by the concentration changing mechanism. Thus, combustion of the sample can be promoted by increasing a combustion-supporting gas concentration, and rapid combustion of the sample can be suppressed by lowering the combustion-supporting gas concentration. Thus, by flexibly changing an amount of combustion in accordance with a type of a sample, such as a flame-retardant sample that is difficult to burn, an explosive sample that is easy to burn, and a mixed sample including a component that burns in a low temperature range and a component that burns in a high temperature range, it is possible to promote combustion, suppress rapid combustion, and thus analyze various samples.

For example, in order to promote the combustion of a flame-retardant sample such as steel or non-ferrous metal ceramics, the combustion-supporting gas is preferably a high-concentration oxygen gas.

The electric furnace is preferably pressurized inside when the sample is heated.

With such a configuration, the combustion of a flame-retardant sample can be promoted by pressurizing the inside of the electric furnace and increasing the combustion-supporting gas concentration, and the rapid combustion of an explosive sample can be suppressed by decreasing the combustion-supporting gas concentration while pressurizing the inside of the electric furnace. Then, it is possible to analyze both the flame-retardant sample and the explosive sample with the same analysis device.

The analysis device preferably further includes a furnace controller configured to control an operation of the electric furnace, in which the furnace controller is configured to heat the electric furnace before the container accommodating the sample is introduced into the electric furnace.

In this configuration, time from the introduction of the container to the combustion of the sample can be made shorter than in a configuration in which the electric furnace is heated after the container accommodating the sample is introduced into the electric furnace. Thus, the analysis takes less time, and the analysis can be more efficient.

The container is preferably introduced into the electric furnace through an opening formed in a side wall, and a driving gas for an opening and closing lid that opens and closes the opening is preferably also used as the dilution gas.

This configuration eliminates the need for preparing a dedicated dilution gas for diluting the combustion-supporting gas.

The combustion-supporting gas and the dilution gas have different viscosity, and thus a viscosity of the mixed gas changes depending on the combustion-supporting gas concentration in the mixed gas. This change in viscosity can affect analysis accuracy.

Therefore, in order to ensure the analysis accuracy, the analysis device preferably further includes a calculator configured to calculate a mass concentration of a component included in the sample on the basis of a light intensity signal outputted from the gas analyzer, and a correction data storage configured to store correction data that associates a target combustion-supporting gas concentration, which is set in advance as a concentration of the combustion-supporting gas in the mixed gas, with a correction coefficient, in which the calculator corrects the mass concentration that has been calculated, using the target combustion-supporting gas concentration and the correction coefficient obtained from the correction data.

In order to suppress the combustion when the amount of combustion of the sample is excessively large, the analysis device preferably further includes a concentration controller configured to control the concentration changing mechanism on the basis of a light intensity signal outputted from the gas analyzer or a concentration of a component included in the sample calculated on the basis of the light intensity signal.

In order to reduce an influence of a use of the dilution gas on the analysis accuracy, the gas analyzer preferably has a non-dispersive infrared detector, and the combustion-supporting gas and the dilution gas preferably have an identical infrared absorption rate.

Further, an analysis method of the present invention is an analysis method using an analysis device including an electric furnace into which a container accommodating a sample is introduced, a gas analyzer configured to analyze a gas generated from the sample that is heated and burned in the electric furnace, and a mixed gas supply path configured to supply, to the electric furnace, a mixed gas obtained by mixing a dilution gas with a combustion-supporting gas that promotes combustion of the sample, the analysis method including changing a concentration of the combustion-supporting gas in the mixed gas, and supplying the mixed gas from the mixed gas supply path to the electric furnace. This analysis method can also have the same effect as the above analysis device.

### Advantageous Effects of Invention

The present invention described above makes it possible to flexibly change the amount of combustion in accordance with the type of sample such as a flame-retardant sample, an explosive sample, a mixed sample, and the like, promote combustion, suppress rapid combustion, and analyze various samples.

### Brief Description of Drawings

FIG. 1 is a schematic diagram of a configuration of an electric furnace of an analysis device according to the present embodiment.
FIG. 2 is a schematic diagram of an overall structure of the analysis device according to the present embodiment.
FIG. 3 is a functional block diagram illustrating a function of a control device according to the present embodiment.
FIG. 4 is a schematic diagram of an overall configuration of an analysis device according to another embodiment.
FIG. 5 is a functional block diagram illustrating a function of a control device according to another embodiment.

### Description of Embodiments

Hereinafter, one embodiment of the present invention will be described with reference to the drawings.

An analysis device 100 according to the present embodiment heats and burns a powdery or bulk solid sample, and analyzes elements such as carbon (C) and sulfur (S) included in the sample from a gas generated by the heating and burning. Examples of the sample include those containing an inorganic material such as steel and non-ferrous metal, those containing an organic material such as coal, and those containing both an inorganic material and an organic material.

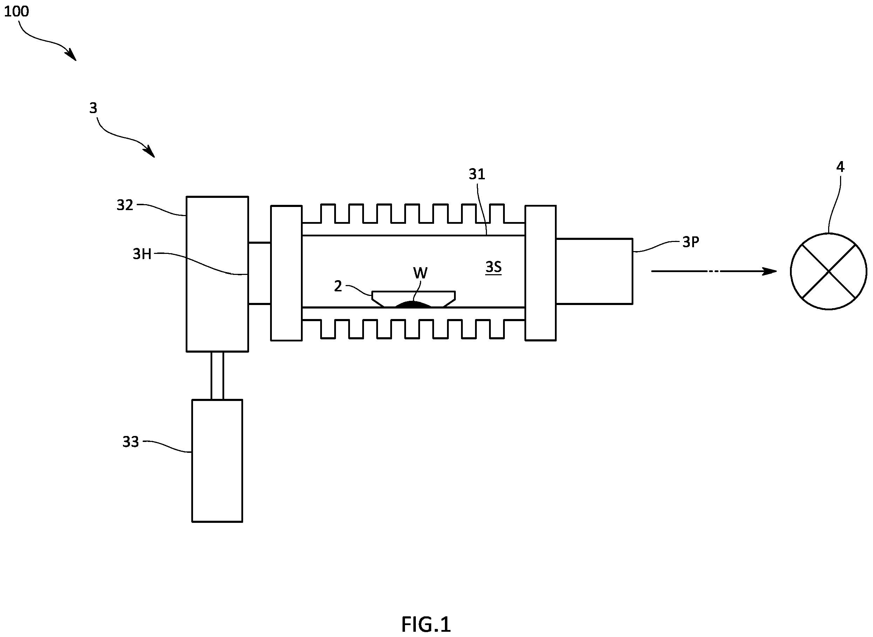

Specifically, as shown in FIG. 1, this analysis device 100 includes an electric furnace 3 in which a container 2 accommodating a sample W is disposed and a gas analyzer 4 that analyzes a gas generated from the sample W heated and burned in the electric furnace 3.

The container 2 accommodates the sample W, for example, in powdery form inside. The container 2 according to the present embodiment has a long shape having an opening at a top, and is specifically a magnetic combustion boat. The shape of the container 2 is not limited to this, and various shapes can be used.

Inside the electric furnace 3, the container 2 not accommodating the sample W is air-baked, or the container 2 accommodating the sample W is heated to heat the sample W to generate gas.

Specifically, as shown in FIG. 1, the electric furnace 3 has a space 3S extending in a horizontal direction from an opening 3H formed in a side wall, and the container 2 is disposed in the space 3S via the opening 3H. The electric furnace 3 includes a furnace body 31 where the container 2 is put in and out, an electric resistor (not shown) that is provided around the furnace body 31 and heats the furnace body 31, and a power supply circuit (not shown) that supplies electric power to the electric resistor and energizes the electric resistor to generate heat.

The furnace body 31 is, for example, a cylindrical ceramic molded body, and has the space 3S that can accommodate the container 2 inside. The opening 3H is formed at one end of the furnace body 31. Further, at the other end of the furnace body 31, a gas outlet 3P for leading the gas generated from the sample W to the gas analyzer 4 is formed. A method for heating the furnace body 31 may be a method in which an electric current is passed through an electric resistance furnace to perform resistance heating (Joule heat generation). In this case, the furnace body 31 includes a conductive metal.

An opening and closing lid 32 is provided at the opening 3H of the furnace body 31. The opening and closing lid 32 moves between a closing position that closes the opening 3H and an opening position that opens the opening 3H, and is driven by an actuator 33 such as an air cylinder, for example.

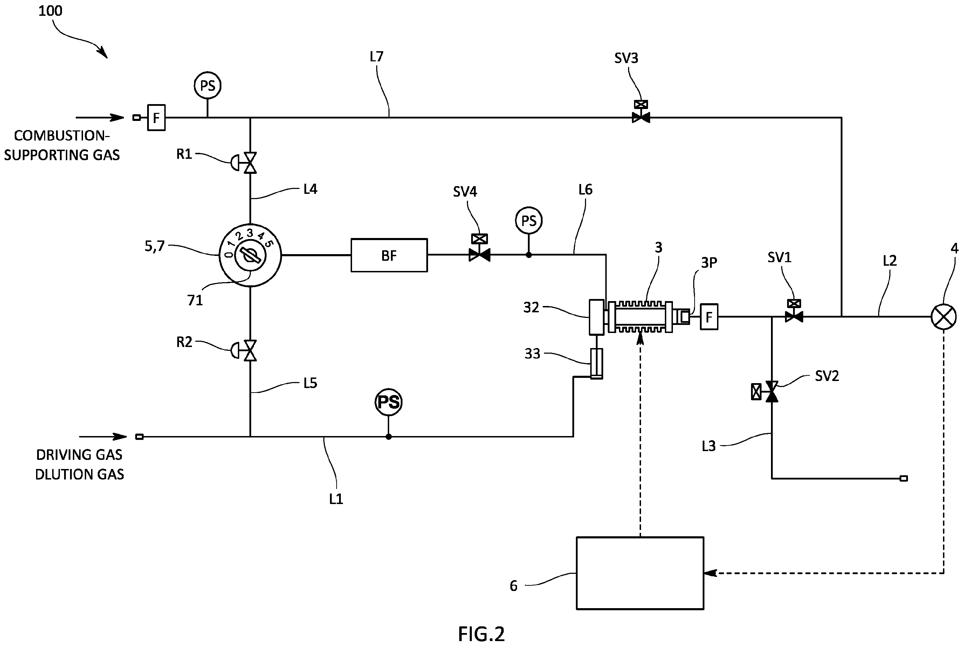

As shown in FIG. 2, the analysis device 100 according to the present embodiment includes a driving gas flow path L1 through which a driving gas for driving the opening and closing lid 32 flows. In this driving gas flow path L1, the driving gas is introduced from a first end opening, and a second end opening is connected to the actuator 33. Here, as the driving gas, for example, nitrogen gas or air is used.

The gas analyzer 4 analyzes the gas generated in the electric furnace 3 and obtains a content value of a target component included in the sample W, and analyzes the gas using, for example, a non-dispersive infrared absorption method (NDIR method). Specifically, the gas analyzer 4 has a non-dispersive infrared detector (not shown) and determines the content value of carbon (C), sulfur (S), and the like included in the sample W by detecting CO₂, CO, SO₂, and the like included in the gas derived from the electric furnace 3. The analysis device 100 may include a plurality of the gas analyzers 4.

As shown in FIG. 2, the gas analyzer 4 is provided on an analysis flow path L2 whose first end opening communicates with the gas outlet 3P of the electric furnace 3 described above, and a first on-off valve SV1 and a filter F are provided upstream of the gas analyzer 4 on the analysis flow path L2.

Further, the analysis device 100 according to the present embodiment is provided with an air release flow path L3 branched from upstream of the first on-off valve SV1 on the analysis flow path L2. The air release flow path L3 is provided with a second on-off valve SV2.

Therefore, as shown in FIG. 2, the analysis device 100 according to the present embodiment includes a combustion-supporting gas flow path L4 through which a combustion-supporting gas that promotes combustion of the sample flows, a dilution gas flow path L5 through which a dilution gas that dilutes the combustion-supporting gas flows, a gas mixer 5 that mixes the dilution gas with the combustion supporting gas and generates a mixed gas, a mixed gas supply path L6 that supplies the mixed gas to the electric furnace 3, and a control device 6 that controls various operations of the analysis device 100.

As shown in FIG. 2, in the combustion-supporting gas flow path L4, the combustion-supporting gas is introduced from a first end opening, and a second end opening is connected to the gas mixer 5. The combustion-supporting gas is, for example, oxygen gas having a concentration of 100% or a high concentration close to 100% (for example, a concentration of 90% or more). The combustion-supporting gas flow path L4 is provided with a regulator R1, which regulates the combustion-supporting gas to a predetermined pressure (for example, 150 kPa). Here, a purging flow path L7 is provided, which is branched from upstream of the regulator R1 in the combustion-supporting gas flow path L4 and connected between the first on-off valve SV1 in the analysis flow path L2 described above and the gas analyzer 4. Here, the combustion support gas is also used as a purge gas. This purging flow path L7 is provided with a third on-off valve SV3.

As shown in FIG. 2, in the dilution gas flow path L5, the dilution gas is introduced from a first end opening, and a second end opening is connected to the gas mixer 5. The dilution gas is a gas that is inert to the combustion-supporting gas or the gas generated by the combustion of the sample, and is, for example, nitrogen gas or argon gas.

Here, the driving gas that drives the opening and closing lid 32 described above is also used as the dilution gas. That is, the dilution gas flow path L5 and the driving gas flow path L1 are common in the upstream. The dilution gas flow path L5 branches at a branch point in a middle and is connected to the gas mixer 5, and the driving gas flow path L1 branches at the same branch point and is connected to the actuator 33.

A regulator R2 is provided downstream of the branch point in the dilution gas flow path L5, and the mixed gas led out from the gas mixer 5 is regulated to a predetermined pressure (for example, 70 kPa).

The gas mixer 5 is connected to the combustion-supporting gas flow path L4 and the dilution gas flow path L5. Specifically, the gas mixer 5 has a first introduction port (not shown) to which the second end opening of the combustion-supporting gas flow path L4 is connected and into which the combustion-supporting gas is introduced, a second introduction port (not shown) to which the second end opening of the dilution gas flow path L5 is connected and into which the dilution gas is introduced, and a lead-out port (not shown) to which a first end opening of the mixed gas supply path L6 is connected and through which the mixed gas is led out.

As shown in FIG. 2, the gas mixer 5 according to the present embodiment has a function as a concentration changing mechanism 7 that changes the concentration of the combustion-supporting gas in the mixed gas.

The concentration changing mechanism 7 is configured to change the concentration of the combustion-supporting gas included in the mixed gas. Specifically, the concentration changing mechanism 7 changes a mixing ratio of the combustion-supporting gas and the dilution gas using, for example, a principle of a capillary flow rate ratio mixing method. Here, an operator can change the concentration of the combustion-supporting gas included in the mixed gas in a stepwise manner by operating a concentration switch 71 such as a knob provided in the concentration changing mechanism 7.

As shown in FIG. 2, the mixed gas supply path L6 has the first end opening connected to the lead-out port (not shown) of the gas mixer 5 and a second end opening communicating with the inside of the electric furnace 3 and supplying the mixed gas led out from the gas mixer 5 into the electric furnace 3.

The mixed gas supply path L6 is provided with a buffer BF capable of storing a predetermined capacity of the mixed gas. Further, a fourth on-off valve SV4, a pressure sensor PS, and the like are provided downstream of the buffer BF in the mixed gas supply path L6. The buffer BF does not have to be provided.

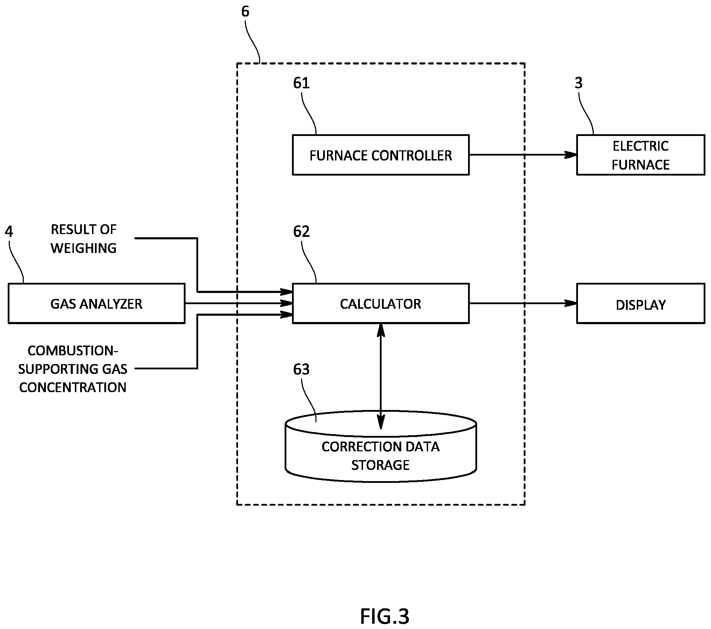

The control device 6 has a CPU, a memory, a display D, an input device, and the like, and as shown in FIG. 3, functions as a furnace controller 61, a calculator 62, a correction data storage 63, and the like on the basis of an analysis program stored in the memory.

Hereinafter, the operation of the analysis device 100 according to the present embodiment will be described together with the explanation of the furnace controller 61, the calculator 62, and the correction data storage 63.

First, the furnace controller 61 controls the power supply circuit (not shown) of the electric furnace 3 to supply electric power to the electric resistor (not shown) and heat the furnace body 31. Here, the container 2 not accommodating the sample W is placed in the electric furnace 3, and the container 2 is air-baked to remove deposits and the like of the container 2. For air baking, another electric furnace arranged in parallel with the electric furnace 3 or another electric furnace outside the device may be used. At this time, the first on-off valve SV1, the third on-off valve SV3, and the fourth on-off valve SV4 are closed, and the second on-off valve SV2 is open.

Then, the sample W after being weighed is accommodated in the container 2 taken out from the electric furnace 3. A result of the weighing of the sample W is input to the control device 6 by the operator, for example.

Next, the furnace controller 61 heats the electric furnace 3 to a predetermined set temperature before the container 2 accommodating the sample W is placed inside the electric furnace 3. The set temperature here can be selected from room temperature to 1,500°C.

Then, in the present embodiment, before the container 2 accommodating the sample W is placed inside the electric furnace 3, a concentration of the combustion-supporting gas included in the mixed gas (hereinafter, a target combustion-supporting gas concentration) is set in consideration of, for example, the components included in the sample W.

Here, as described above, the operator can set and change the target combustion-supporting gas concentration by the concentration changing mechanism 7. For example, when analyzing a flame-retardant sample that is difficult to burn, the operator operates the concentration changing mechanism 7 so as to set the target combustion-supporting gas concentration to a high level (for example, a concentration of 100%).

Further, when analyzing an explosive sample that burns easily or a mixed sample that includes a component that burns in a low temperature range and a component that burns in a high temperature range, the operator operates the concentration changing mechanism 7 so as to set the target combustion-supporting gas concentration low.

In the present embodiment, at least when the target combustion-supporting gas concentration is set high, the mixed gas is supplied into the electric furnace 3, and thus the inside of the electric furnace 3 is in a pressurized state. However, the inside of the electric furnace 3 does not have to be in a pressurized state.

On the other hand, when the target combustion-supporting gas concentration is set low, the inside of the electric furnace 3 does not have to be pressurized and may be, for example, depressurized.

With the target combustion-supporting gas concentration set in this way and the furnace controller 61 adjusting the temperature of the electric furnace 3 to a predetermined set temperature, the third on-off valve SV3 is opened, the gas analyzer 4 is purged, and then the third on-off valve SV3 is closed. Then, the opening and closing lid 32 is opened, the container 2 accommodating the sample W is placed in the electric furnace 3, and the opening and closing lid 32 is closed. Next, the second on-off valve SV2 is closed, and the first on-off valve SV1 and the fourth on-off valve SV4 are opened.

As a result, the sample W is burned in the electric furnace 3 to generate a gas, and the gas is guided to the gas analyzer 4. Then, the gas analyzer 4 outputs a light intensity signal corresponding to a content value of a target component such as C or S included in the gas to the calculator 62, and the calculator 62 calculates a mass concentration of the target component using this light detection signal and the result of the weighing of the sample W.

Here, when the sample W including both the component that burns in the low temperature range and the component that burns in the high temperature range is analyzed, for example, the target combustion-supporting gas concentration is set low until a predetermined time elapses after the container 2 accommodating the sample W is placed in the electric furnace 3, and the setting is changed such that the target combustion-supporting gas concentration becomes high after a lapse of the predetermined time, for example.

By this change of the setting of the combustion-supporting gas, the mass concentration of the component that burns in the low temperature range is calculated before the elapse of the predetermined time, and the mass concentration of the component that burns in the high temperature range is calculated after the elapse of the predetermined time.

In the present embodiment, the correction data storage 63 set in a predetermined area of the memory stores correction data that associates the target combustion-supporting gas concentration with a correction coefficient. Then, for example, the target combustion-supporting gas concentration set by the operator is inputted into the control device 6, and then the calculator 62 acquires the correction coefficient using the input target combustion-supporting gas concentration and the correction data, corrects the calculated mass concentration using the correction coefficient, and outputs the corrected mass concentration to a display or the like.

Examples of such correction data include a calculation formula for calculating a correction coefficient using the target combustion-supporting gas concentration, a viscosity of the combustion-supporting gas, a viscosity of the dilution gas, and the like as parameters, and a table in which the target combustion-supporting gas concentration and the correction coefficient are linked to each other.

The analysis device 100 configured in this way allows the concentration of the combustion-supporting gas in the mixed gas to be changed by the concentration changing mechanism 7. Thus, in analysis of a flame-retardant sample such as metal or ceramic, for example, combustion can be promoted by increasing the target combustion-supporting gas concentration. For example, in analysis of an explosive sample such as an organic material, rapid combustion can be suppressed by lowering the target combustion-supporting gas concentration. Therefore, both a flame-retardant sample and an explosive sample can be analyzed using one same analysis device 100.

Further, in the analysis of an explosive sample, an amount of gas generated by combustion of the sample can be reduced by setting the target combustion-supporting gas concentration low to suppress rapid combustion. As a result, a peak of the signal detected by the gas analyzer becomes gentle, and thus more accurate analysis is possible than when the peak of the detected signal is steep if the same resolution of the detector is the same.

In addition, as described above, suppressing rapid combustion of an explosive sample can prevent the sample from scattering, which also leads to accurate quantitative analysis and a reduction in dirt in the electric furnace 3.

Furthermore, in the analysis of the mixed sample including the component that burns in the low temperature range and the component that burns in the high temperature range, the target combustion-supporting gas concentration is set low at a start of the combustion, and the target combustion-supporting gas concentration is changed to be high in a middle. Then, the component that burns in the low temperature range and the component that burns in the high temperature range can be separated and analyzed. Therefore, for example, a mixed sample including an inorganic material and an organic material can be separated and analyzed with high accuracy.

Furthermore, for example, a high-concentration oxygen gas with a concentration of 100% is used as the combustion-supporting gas, and the inside of the electric furnace 3 is pressurized when the target combustion-supporting gas concentration is set high. Thus, the combustion of the sample W can be further promoted in the analysis of the sample W or the like that burns in the high temperature range.

Further, using the driving gas also as the dilution gas eliminates the need for preparing a dedicated dilution gas for diluting the combustion-supporting gas and makes it possible to use the flow path branched from the existing driving gas flow path L1 as the dilution gas flow path L5. The flow paths can be therefore configured simply.

In addition, because the calculator 62 corrects the calculated mass concentration of the target component using the correction coefficient, a difference in viscosity between the combustion-supporting gas and the dilution gas affects less on the analysis accuracy.

The present invention is not limited to the above embodiments.

For example, in the above embodiment, a case where the operator sets the target combustion-supporting gas concentration has been described; however, the target combustion-supporting gas concentration may be automatically controlled by using, for example, the control device 6 or another computer.

In this case, the concentration changing mechanism 7 may be configured to receive a control signal and change the target combustion-supporting gas concentration in a stepwise manner or a stepless manner.

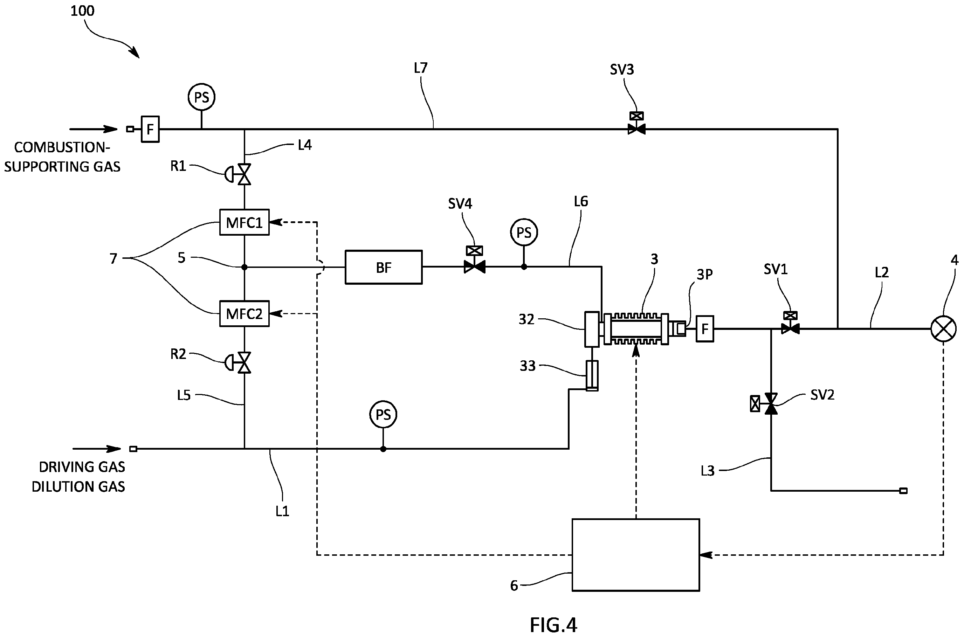

Further, in the above embodiment, a case where the gas mixer 5 has a function as the concentration changing mechanism 7 has been described; however, the concentration changing mechanism 7 may be configured using one or a plurality of flow rate controllers MFC provided separately from the gas mixer 5 as shown in FIG. 4.

A specific example of the concentration changing mechanism 7 is a configuration having a first flow rate controller MFC1 as a mass flow controller provided in the combustion-supporting gas flow path L4 and a second flow rate controller MFC2 as a mass flow controller provided in the dilution gas flow path L5 as shown in FIG. 4.

With such a configuration, for example, the control device 6 can control the target combustion-supporting gas concentration by controlling the first flow rate controller MFC1 and the second flow rate controller MFC2, and the target combustion-supporting gas concentration can be controlled automatically.

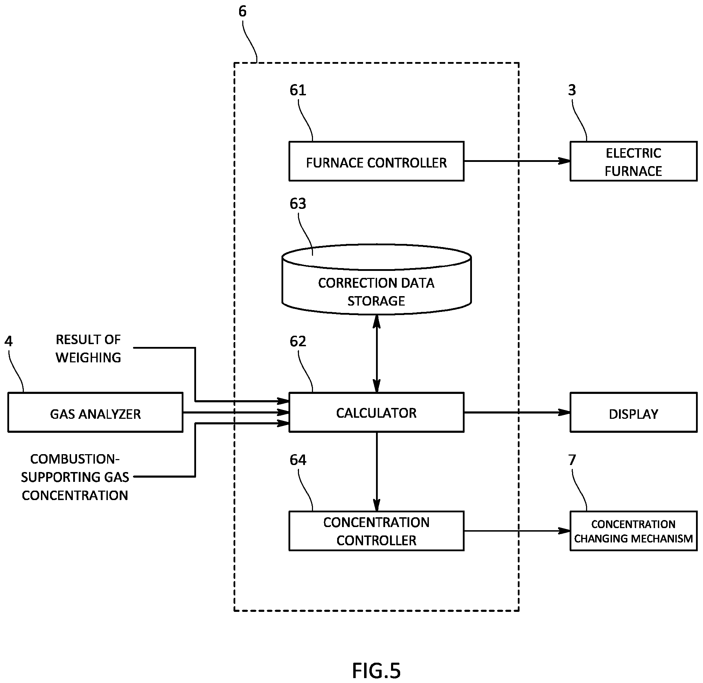

As described above, in the configuration in which the target combustion-supporting gas concentration is automatically controlled, an aspect of the control device 6 further has a function as the concentration controller 64 controlling the concentration changing mechanism 7, as shown in FIG. 5.

The concentration controller 64 here is configured to control the concentration changing mechanism 7 on the basis of the concentration of the component included in the sample calculated by the calculator 62. Specifically, the concentration controller 64 controls the concentration changing mechanism 7 to decrease the target combustion-supporting gas concentration when the concentration calculated by the calculator 62 exceeds a preset upper limit value.

This configuration can suppress the combustion of the sample W when an amount of combustion of the sample W is large and the concentration calculated by the calculator 62 exceeds the upper limit value.

Further, the concentration controller 64 preferably controls a gas mixing mechanism to increase the target combustion-supporting gas concentration when the concentration calculated by the calculator 62 falls below a preset lower limit value.

This configuration can promote the combustion of the sample W when the amount of combustion of the sample W is small and the concentration calculated by the calculator 62 falls below the lower limit value.

Note that the concentration controller 64 may be configured to control the concentration changing mechanism 7 on the basis of the light intensity signal outputted from the gas analyzer 4, or may be configured to perform feedback control of the target combustion-supporting gas concentration using the light intensity signal or the concentration calculated by the calculator 62.

In the case of the analysis of a sample including both a component that burns in the low temperature range and a component that burns in the high temperature range, in the above embodiment, the method in which the target combustion-supporting gas concentration is set low at the beginning and changed to be high in the middle was described above. However, instead of or in addition to this change of setting of the target combustion-supporting gas concentration, the set temperature of the electric furnace may be changed in the middle.

The gas analyzer is not limited to the analyzer using the NDIR method described in the above embodiment, but may be an analyzer using a Fourier transform infrared spectroscopy (FTIR) method or an analyzer using a non-dispersive ultraviolet spectroscopy (NDUV) method.

In addition, various modifications or combinations may be made to the embodiments without contradicting the gist of the present invention.

### Reference Signs List

- 100: analysis device
- 2: container
- 3: electric furnace
- 4: gas analyzer
- 5: gas mixer
- 6: control device
- 7: concentration changing mechanism
- L4: combustion-supporting gas flow path
- L5: dilution gas flow path
- L6: mixed gas supply path

### Industrial Applicability

The present invention can promote the combustion of the sample in the high temperature range and also reduces the amount of combustion of the sample in the low temperature range.

## Claims

1. An analysis device comprising:
an electric furnace into which a container accommodating a sample is introduced;
a gas analyzer configured to analyze a gas generated from the sample that is heated and burned in the electric furnace;
a mixed gas supply path configured to supply, to the electric furnace, a mixed gas obtained by mixing a dilution gas with a combustion-supporting gas that promotes combustion of the sample; and
a concentration changing mechanism configured to change a concentration of the combustion-supporting gas in the mixed gas.

2. The analysis device according to claim 1, wherein the combustion-supporting gas is a high-concentration oxygen gas.

3. The analysis device according to claim 1, wherein the electric furnace is pressurized inside when the sample is heated.

4. The analysis device according to claim 1, further comprising a furnace controller configured to control an operation of the electric furnace, wherein the furnace controller is configured to heat the electric furnace before the container accommodating the sample is introduced into the electric furnace.

5. The analysis device according to claim 1, wherein
the container is introduced into the electric furnace through an opening formed in a side wall, and
a driving gas for an opening and closing lid that opens and closes the opening is also used as the dilution gas.

6. The analysis device according to claim 1, further comprising:
a calculator configured to calculate a mass concentration of a component included in the sample on a basis of a light intensity signal outputted from the gas analyzer; and
a correction data storage configured to store correction data that associates a target combustion-supporting gas concentration with a correction coefficient, the target combustion-supporting gas concentration being set, in advance, as a concentration of the combustion-supporting gas in the mixed gas,
wherein the calculator corrects the mass concentration that has been calculated, using the target combustion-supporting gas concentration and the correction coefficient obtained from the correction data.

7. The analysis device according to claim 1, further comprising a concentration controller configured to control the concentration changing mechanism on a basis of a light intensity signal outputted from the gas analyzer or a concentration of a component included in the sample calculated on a basis of the light intensity signal.

8. The analysis device according to claim 1, wherein
the gas analyzer has a non-dispersive infrared detector, and
the combustion-supporting gas and the dilution gas have an identical infrared absorption rate.

9. An analysis method using an analysis device including an electric furnace into which a container accommodating a sample is introduced, a gas analyzer configured to analyze a gas generated from the sample that is heated and burned in the electric furnace, and a mixed gas supply path configured to supply, to the electric furnace, a mixed gas obtained by mixing a dilution gas with a combustion-supporting gas that promotes combustion of the sample, the analysis method comprising:
changing a concentration of the combustion-supporting gas in the mixed gas; and
supplying the mixed gas from the mixed gas supply path to the electric furnace.
